# EUROPEAN PATENT APPLICATION

(11) **EP 0 567 187 A1**
(43) Date of publication of application: **27.10.1993**
(21) Application number: 93201091.1
(22) Date of filing: 15.04.1993
(51) Int. Cl.: G01N 33/545, G01N 33/58

(54) **An immunoassay and test kit**

(30) Priority: 21.04.1992 GB 9208548
(71) Applicant: KODAK LIMITED, Harrow, Middlesex HA1 4TY (GB); Johnson & Johnson Clinical Diagnostics, Inc., Rochester New York 14650 (US)
(72) Inventor: Bamford, Clement Henry, c/o Kodak Limited, Harrow,Middlesex, HA1 4TY (GB); Al-Lamee, Kadem Gayad, c/o Kodak Limited, Harrow,Middlesex, HA1 4TY (GB); Purbrick, Malcolm Donald, c/o Kodak Limited, Harrow,Middlesex, HA1 4TY (GB); Wear, Trevor John, c/o Kodak Limited, Harrow,Middlesex, HA1 4TY (GB)
(74) Representative: Mercer, Christopher Paul

(57) **Abstract**

An immunoassay is carried out using a fiber-forming or film-forming polymer as a capture support. The polymer is a polyamide, polyurethane or polyurea which has activating groups attached to the polymer through the nitrogen atom of the amide, urethane or urea groups of the polymer. The activating groups are reactive with amine or thiol groups of various compounds, such as biologically useful compounds. A test kit useful in the immunoassay includes the capture support and detectably labelled antibodies directed to an analyte of interest.

## Description

The invention relates to an immunoassay and test kit for the detection of an analyte.

A variety of immunoassay techniques are known in which a component of an immune complex formed during the assay is covalently attached to a polymer support. The support on which the complex is immobilised can take different forms. For example, one preferred form of support is a porous membrane.

Clin. Prod. Rev., 4, 1985, 33-41 describes the use of a commercially available microporous membrane known as IMMOBILON™ derived from a polyvinylidene difluoride polymer. An example of its use in an enzyme-linked immunosorbent assay (ELISA) is given.

There is a need for alternative polymer support materials for immunoassays. There is a particular need for polymer supports which can enhance the sensitivity of an assay.

The invention provides an immunoassay for the detection of an analyte which comprises
(A) forming a detectably labeled immune complex comprising the analyte on a support, and
(B) detecting the labeled immune complex on the support as an indication of the presence or absence of the analyte,
characterised in that the support is derived from a fibre-forming or film-forming polyamide, polyurethane or polyurea polymer comprising activating groups attached to said polymer through the nitrogen atom of the amide, urethane or urea groups of said polymer, the activating groups being capable of reaction with an amino or thiol group of a compound containing an amino or thiol group to effect covalent attachment of the compound to the polymer, and the detectably labeled immune complex is covalently bound to the polymer by the formation of a link by reaction between the activating group of the polymer and a component of the immune complex.

The invention also provides a test kit for an immunoassay for the detection of an analyte comprising:
(a) a water-insoluble article comprising a porous membrane having at least first and second opposing outer surfaces and having an anti-analyte antibody covalently bound thereto, and
(b) detectably labelled antibodies directed to the analyte,
the kit characterised in that the porous membrane is derived from a fibre-forming or film-forming polyamide, polyurethane or polyurea polymer comprising activating groups attached to said polymer through the nitrogen atom of the amide, urethane or urea groups of said polymer, the activating groups being capable of reaction with an amino or thiol group of a compound containing an amino or thiol group to effect covalent attachment of the compound to the polymer and the anti-analyte antibody is covalently bound to the polymer by the formation of a link by reaction between the activating group of the polymer and the antibody.

Suitable polymers can be prepared by modifying any polyamide, polyurethane or polyurea having fibre-forming or film-forming properties. The polymer may be an elastomer and, in a preferred embodiment of the invention, a polyetherurethane is employed. Examples of suitable commercially available polymers from which polymers of the support can be prepared include, but are not limited to, BIOMER™, PELLETHANE™, TECOFLEX™ and ESTANE™ polymers.

Groups capable of coupling with an amino or thiol group-containing compound e.g. by the formation of an amide or thioether link, respectively, are known.

Preferred activating groups include an isocyanate group, an imidazolyl carbamate group, a 1-methyl-2-pyrydyl group or a group having the formula - COOZ wherein Z is an electron-withdrawing group. Functional groups are classified as electron-withdrawing groups relative to hydrogen, e.g. -NO₂ and -I groups draw electrons to themselves more than a hydrogen atom occupying the same position in the molecule, J. March, Advanced Organic Chemistry, 2nd edition, McGraw Hill, p.20 and 246. Specific examples of Z groups include N-succinimido, benzylidene aniline, pentafluorophenyl, 4-nitrophenyl, 4-cyanophenyl, 4-alkylsulphonylphenyl, acyl, 4-acylphenyl, 4-dialkylaminocarbonylphenyl, 4-alkoxycarbonylphenyl and 4-alkoxysulphonylphenyl.

Preferably, the polymer of the invention comprises units having the formula 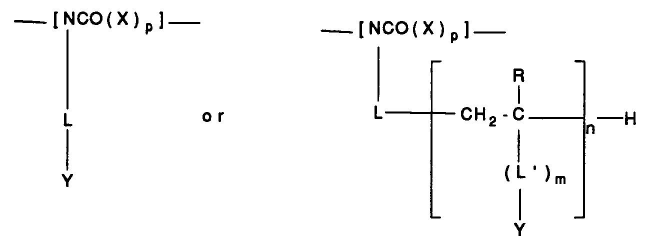
wherein -[NCO(X)ₚ]- in which X is -O- or -NH- and p is 0 or 1, is an amide, urethane or urea group in the polymer backbone, L and L' are each independently a linking group, R is hydrogen or alkyl, Y is an activating group, m is 0 or 1 and n is an integer from 10 to 150, preferably from 30 to 120.

L and L' together with the atoms linking them serve to space the activating group Y away from the polymer backbone. Each of L and L' may comprise one or more divalent hydrocarbon groups such as substituted or unsubstituted alkylene and arylene groups which are connected or terminated with heteroatoms or heteroatom-containing groups such as - O-, -N- , -S-, -NHCO-, -COO- and -CO-. Preferably, L comprises a chain of from 4 to 50 atoms separating the activating group or the activating group-containing moiety from the polymer backbone.

Specific examples of L and L' groups are shown in the following schematic representations of polyurethane polymers of the invention wherein the term "Polymer" is used to indicate the remainder of the polyurethane polymer which contains further urethane groups similarly substituted: 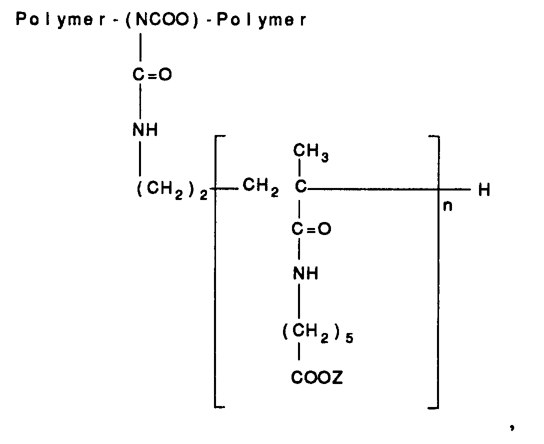 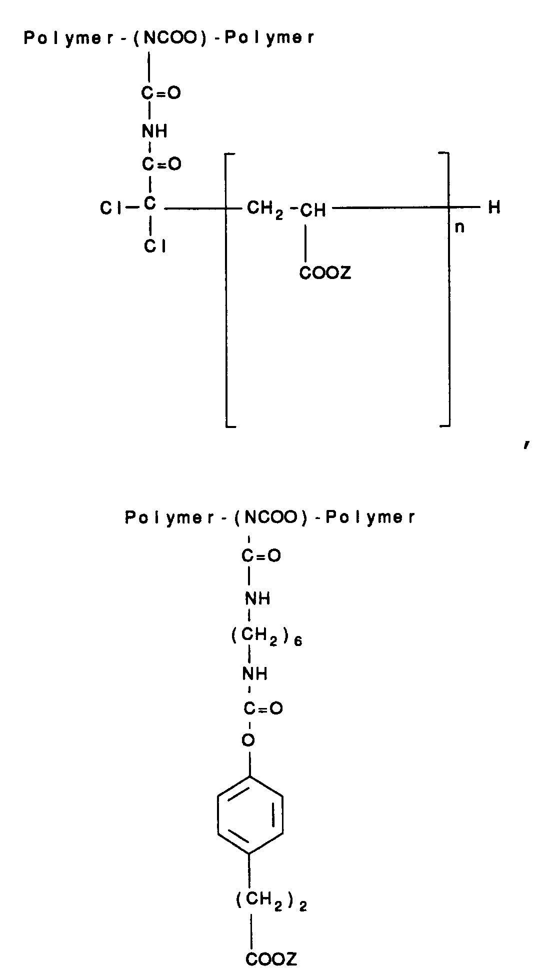 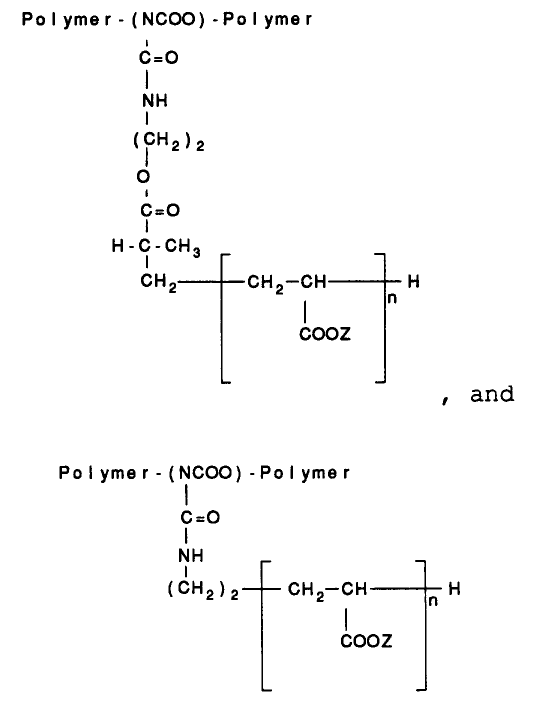
The activating group reacts directly with the amino group-containing compound. Preferably, such reaction will take place under physiological reaction conditions.

A number of synthetic methods are available for preparing the polymers of the invention.

One method comprises reacting a fibre-forming or film-forming polyamide, polyurethane or polyurea with a haloisocyanate or an ethylenically unsaturated isocyanate and subsequently grafting an ethylenically unsaturated monomer comprising an activating group onto the product.

Examples of haloisocyanates include haloalkyl and haloacetyl isocyanates e.g. 2-chloroethyl isocyanate and trichloroacetyl isocyanate. Examples of ethylenically unsaturated isocyanates include isocyanato acrylate monomers e.g. isocyanatoethyl methacrylate.

Examples of ethylenically unsaturated monomers comprising an activating group include N-acryloyloxy-succinimide and the succinimide ester of 6-methacrylamidocaproic acid.

Another method of preparing a suitable polymer comprises reacting a fibre-forming or film-forming polyamide, polyurethane or polyurea with a diisocyanate. The free isocyanate group of the reaction product is an activating group. If desired, the product containing free isocyanate groups may be reacted with a hydroxy-containing reactive ester. Alternatively, the product containing free isocyanate groups may be reacted with an alkanolamine or other amino alcohol, or a diol, to produce a hydroxylated or carboxylated polymer which may subsequently be activated.

Examples of diisocyanates include alkylene and arylene diisocyanates, e.g. hexamethylene diisocyanate and 2,4-tolylene diisocyanate.

Examples of hydroxy-containing reactive esters include hydroxyalkyl, hydroxyaryl, hydroxyalkaryl and hydroxyaralkyl reactive esters, e.g. N-[3-(4-hydroxyphenyl)-propionyloxy]-succinimide.

Examples of compounds used to convert the free isocyanate groups of the polymer to hydroxylated forms include alkanolamines such as ethanolamine, 6-amino-1-hexanol and glucamine, and diols such as poly(ethylene glycol).

Examples of compounds used to convert the free isocyanate groups of the polymer to carboxylated forms include amine group-containing carboxylic acids such as 6-aminocaproic acid.

Examples of components used for activating the hydroxylated polymer are 1,1'-carbonyldiimidazole (CDI) and 2-fluoro-1-methyl pyridinium toluene 4-sulphonate (FMP).

The methods described above may be carried out in solution such that the polymer is dissolved prior to reaction. In this way, an activated polymer is formed which may subsequently be shaped into the desired fibrous or film form.

Alternatively, the polymer in solid form e.g. in fibrous or film form may be treated with solutions of the reactants so that only the surface of the polymer is activated.

In a preferred embodiment of the invention, the support is a fibrous membrane. The fibres may be produced by electrostatic spinning in accordance with the teaching of GB-A-1 530 990. The activated polymer may be spun into fibres. Alternatively, a polymer may be spun into fibres and then modified by the attachment of activating groups.

In the electrostatic spinning process, the fibres are collected as a porous mat on a suitably located receiver. In this way, a substrate coated with a layer of the fibres can be produced. Alternatively, the fibrous mat can be stripped from the receiver.

The fibrous product can be produced in a variety of shapes. For example, by using a cylindrical receiver, a tubular product can be made.

The fibres obtained by the electrostatic spinning process are thin and can be of the order of 0.1 to 25 µm in diameter. Fibre diameters of 0.5 to 10µm, especially 1.0 to 5 µm may be preferred.

The polymer may be conveniently spun from solution. Suitable solvents include dimethylformamide, N,N-dimethylacetamide, dichloromethane and methyl ethyl ketone. Solvent mixtures may be preferred, such as a mixture of N,N-dimethylformamide and methyl ethyl ketone (1.45:1 weight ratio). The concentration of the polymer in solution will depend upon the amount required to provide adequate fibre properties and will be influenced by the need to produce a liquid of appropriate viscosity and speed of fibre hardening. For example, a preferred concentration when using BIOMER™ polymer, a commercially available poly(etherurethaneurea) having a molecular weight in the region of 60,000, dissolved in N,N-dimethylacetamide is from 10 to 20% w/w, for example, 16% w/w.

Any convenient method may be employed to bring the polymer solution into contact with the electrostatic field for spinning. For example, the solution may be supplied to an appropriate position in the electrostatic field by feeding it to a nozzle from which it is drawn by the field to form fibres. The solution may be fed from a syringe reservoir to the tip of a grounded syringe needle, the tip being located at an appropriate distance from an electrostatically charged surface. Upon leaving the needle, the solution forms fibre between the needle tip and the charged surface. The electrostatic potential employed may be conveniently from 10 to 100 Kv, preferably from 10 to 50 Kv.

The pore size and porosity of the fibrous product may be controlled, for example, by varying such parameters as the diameter of the fibres and their density of deposition.

Typically, the fibrous product comprises a network of very fine fibres having a diameter of approximately 1 µm. The fibres are melded at many junction points and enclose irregular holes or pores with a typical dimension in the range from 0.5 to 10 µm. The overall surface area of the fibres is extremely large. For example, 1 g of the fibrous material may have a total surface area of approximately 4 m².

The polymeric supports described above may be used for a variety of immunoassay procedures and analytes. The analytes include the wide range of antigenic materials to which antibodies can be raised.

Examples of the component of the immune complex covalently attached to the support include an antibody e.g. an anti-analyte antibody, a nonantibody protein e.g. protein A and the analyte itself.

In a preferred embodiment of the invention the immunoassay comprises the steps of
(i) contacting an aqueous specimen suspected of containing the analyte with the support having an anti-analyte antibody covalently bound thereto, and
(ii) prior to, simultaneously with or subsequent to the contact in step (i), labelling the analyte with a detectably labelled antibody.

When the polymeric support is a porous membrane, uncomplexed materials can be separated from the immobilised immune complex by washing or draining through the membrane.

If desired, the polymeric support can be coated with proteins known in the art to reduce non-specific interactions.

Detectably labelled antibodies include antibodies having a moiety covalently attached thereto or otherwise associated therewith which can be directly or indirectly detected visually or by using spectrophotometer, radiometric or other suitable equipment and procedures. Indirect detection can be accomplished by reacting the moiety on the antibody one or more times with suitable reagents to render the resulting product directly detectable..

Useful labels are well known and include enzymes, radioisotopes, and specific binding materials such as biotin, avidin, lectins and sugars. Preferably, contact with the labelled antibody occurs after complex formation with the insolubilised antibody on the membrane. The resulting bound sandwich complex is thus formed of a specific antigen and two antibodies directed to different epitopic sites thereof, one antibody insolubilised, the second antibody being labelled for detection.

Detection of the immune complex on the support may be accomplished using standard detection equipment and procedures. Where the label is an enzyme, the bound complex may be further contacted with a composition which will provide a dye (chromogen or fluorogen) in the presence of the enzyme. Such a composition generally includes one or more reagents which are substrates for the enzyme.

Where peroxidase is the label, a number of suitable dye-forming compositions are known comprising a substrate or substrate-forming reactant as well as dye-forming reactants.

In a preferred embodiment of the invention, the polymeric support is a porous membrane disposed or mounted in a water-insoluble article having a water-insoluble frame or structure for holding the membrane. Such articles are often identified in the art as test devices and many forms of such devices are known. A preferred test device comprises a water-insoluble shell having one or more test wells therein, each of which can accommodate a sample of an analyte-containing specimen and appropriate reagents.

The preparation of support polymers is illustrated as follows. In the following Preparations, -(NHCOO)- represents a urethane group in the polyurethane polymer used. BIOMER™ polymer is a commercially available poly(etherurethaneurea) having the structure: 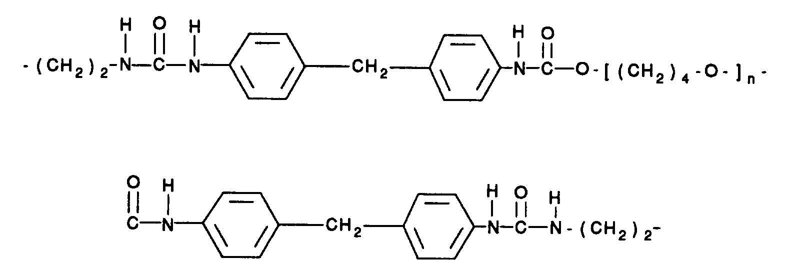
The molecular weight (MnW) of the polymer is about 60,000.

### Preparation 1

The synthesis of a pre-activated polyetherurethane was effected in the three steps described below.

### 1. Functionalisation of polyetherurethane.

A polyetherurethane (BIOMER™, Ethicon, Someville, NJ: 30 g) was dissolved in N,N-dimethylacetamide (DMAC) (50 ml). 2-Chloroethyl isocyanate (4 ml) was added to the resultant solution. The reaction mixture was kept for 3 days at room temperature and then precipitated into water. After precipitation the polymer was filtered off, washed carefully with water and then dried in a vacuum oven.

The reaction of the polyetherurethane with 2-chloroethyl isocyanate gives an allophanate product (I), as represented by the following equation: 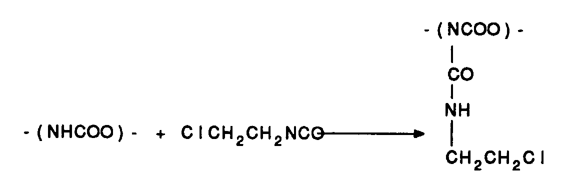
Chlorine analysis of the functionalised polyetherurethane (I) produced: % Cl = 1.12 2. Synthesis of the spaced active ester monomer, the N-succinimido ester of 6-methacrylamidocaproic acid (II).

6-Aminocaproic acid (26.2 g, 0.2 moles) was dissolved in a solution of sodium hydroxide (8.0 g) in water (25 ml) . TOPANOL OC™, a commercially available surfactant from ICI comprising 4-methyl-2,6-tertiary-butyl phenol, was added, and the solution cooled to -10°C. A solution of methacryloyl chloride (20.8 g, 0.2 moles) in dioxane (15 ml) was then added simultaneously with a solution of sodium hydroxide (8.0 g) in water (20 ml) over a period of 1 hour. The latter two solutions had been cooled in an ice-bath prior to their addition. On completion of the addition, the reaction was stirred for a further 2 hours at -10°C. The reaction mixture was then left to stand overnight in the refrigerator.

After standing overnight, the reaction mixture was adjusted to pH 4 with dilute hydrochloric acid. The solution was concentrated using a rotary evaporator, and the residue extracted with ethyl acetate. The combined extracts were washed with water and dried over magnesium sulphate. The solution was filtered and the solvent removed at the rotary evaporator.

Ethyl acetate/petroleum ether (60-80°C) was added to the (oil) residue. This resulted in separation into an oily layer and a cloudy solvent layer. The solution was shaken vigorously and, upon settling, the cloudy solvent layer was removed and retained. Further ethyl acetate/petroleum ether (60-80°) was added and the above successively repeated until the oily layer was no longer observed. The product was obtained by cooling the combined extracts in an ice-salt bath and scratching (yield: 22.6 g, 70%).

6-Methacrylamidocaproic acid (10 g, 0.05 moles) and N-hydroxysuccinimide (5.75 g, 0.05 moles) were placed in a three-necked flask that was fitted with a magnetic stirrer, air condenser (with calcium chloride guard tube) and a dropping funnel. Dichloromethane (50 ml) and tetrahydrofuran (10 ml) and 4-methylaminopyridine (4-DMAP) (0.12 g) were added, and the solution was stirred in an ice bath. A solution of dicyclohexylcarbodiimide (DCCI) (11.5 g) in dichloromethane (20 ml) was added dropwise. The urea precipitated in due course and the reaction was allowed to run overnight.

The solid (urea) precipitate was filtered off and washed with dichloromethane. The combined washings and filtrate were stripped on the rotary evaporator. The residual oil was dissolved in acetonitrile and the solution was cooled in the refrigerator for 2 hours. The small amount of urea which had precipitated was filtered off and the solvent was then removed under vacuum. The remaining oil was dissolved in ethyl acetate. The solid product precipitated on standing in an ice-salt bath, and was filtered and dried.

Analytical and spectroscopic data were consistent with the required structure (II).
Yield: 4.29 g (29%); mp 77.5 °C.

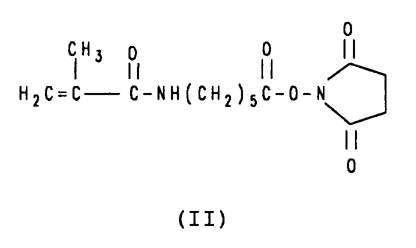

### 3. Grafting monomer (II) to functionalised polyetherurethane.(I)

The functionalised polyetherurethane (I) (7.56 g) was dissolved in pure DMAC (60 ml). A solution of 1.56 g of monomer (II) in DMAC (5 ml) was then added to this, together with a solution of 0.053 g of Re₂ (CO)₁₀ in DMAC (2 ml). The reaction mixture was then degassed under vacuum and sealed off. The grafting was carried out photochemically (λ= 365 nm) for 7 hours at room temperature in accordance with the teaching of C.H. Bamford in Reactivity Mechanism & Structure in Polymer Chemistry ed. A.D. Jenkins & A. Ledwith, John Wiley 1974, Chapter 3. The polymer solution was then precipitated into a mixture of a very dry diethyl ether/ethyl acetate (9:1). After precipitation, the polymer product was filtered off and washed with diethyl ether, vacuum dried and weighed. The weight increase - corresponding to grafting of the polymer of (II) onto (I) to generate the pre-activated polyetherurethane (III) - was 8.14%.

The photochemically initiated grafting reaction is represented in the following equation. 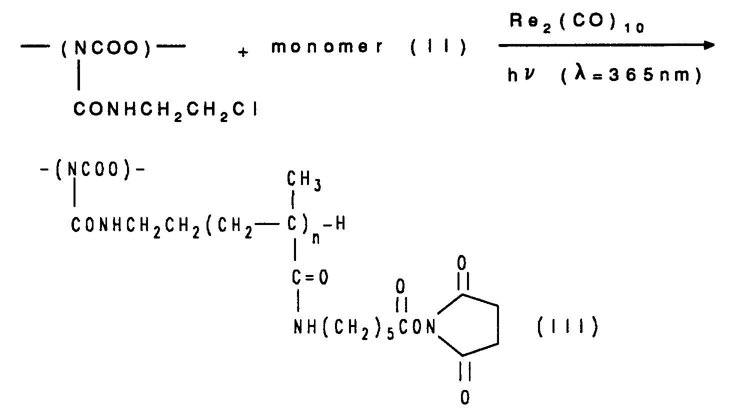
Using the procedure described above, the functionalized polymer (I) was also reacted with N-acryloxy succinimide to provide another activated polymer.

### Covalent coupling of protein

The pre-activated polymer (III) was dissolved in bulk DMAC to obtain a concentration suitable for electrostatic spinning (16% w/w). The solution was spun at minimum humidity following the procedure given in GB-A-1 530 990 to produce the required sheet of fibrous pre-activated polyetherurethane.

The sheet was cut into strips measuring 2 x 1 cm. Samples of the strips were immersed in a solution of radiolabelled Protein A (1.0 ml, 1 mg Protein A/ml 0.1 molar sodium hydrogen carbonate buffer, pH 8). The strips were left to stand for 2 hours at room temperature. The strips were then removed, washed first in excess buffer and then in deionised water before blotting dry on a filter paper.

The strips were allowed to stand in a solution of sodium dodecyl sulphate (SDS) (5 ml, 2% by weight) for one hour at room temperature. They were then washed with deionised water and dried.

Each strip was then counted for one minute in a scintillation counter and compared with a reference to determine the quantity of Protein A covalently bound to the polyetherurethane.

The results showed that Protein A was coupled successfully to the polymer at a level of about 34 mg/m².

The above procedure was repeated using radiolabelled human IgG (Sigma Chemical, 1 mg/ml) instead of Protein A. The results showed that the human IgG was coupled successfully to the polymer also at a level of about 34 mg/m².

The binding activity of the Protein A coupled to the polymer was assessed as follows.

Four strips of the pre-activated polyetherurethane (2 x 1 cm each) were added to a solution of Protein A (2.5 ml, 1.0 mg/ml) in coupling buffer, 0.1 molar sodium hydrogen carbonate, pH 8. The strips were incubated for two hours at room temperature, washed with coupling buffer followed by water and blotted dry on filter paper.

The strips were then added to a blocking reagent (1 molar ethanolamine pH 8, 5 ml) and left to stand for one hour at room temperature. The strips were washed with water and stored in 0.15 molar PBS at approximately 4°C.

The strips of polyetherurethane having Protein A coupled thereto were placed in a solution of radiolabelled human IgG (1.0 mg/ml, 1 ml) for one hour at room temperature. The strips were removed, washed with water and placed in 0.15 molar PBS containing 0.2% TWEEN™ 20 nonionic surfactant (5 ml) for five minutes to remove non-specifically bound protein. The strips were rewashed with water and blotted dry on filter paper. Each strip was counted for one minute using a scintillation counter to provide a measure of specific binding.

Other strips of (unlabeled) polyetherurethane having Protein A coupled thereto were incubated for one hour in a solution of radiolabelled Protein A made up in 0.15M PBS/TWEEN™ 20 nonionic surfactant as above to provide a measure of non-specific binding of protein.

Reference strips were prepared by adsorbing a known quantity of radiolabelled human IgG (1 mg/ml) on the polyetherurethane and counted.

The results showed that the specific binding of human IgG to the polyetherurethane having Protein A coupled thereto was about 92 mg/m². The non-specific binding of protein to the equivalent sample was found to be about 18 mg/m².

### Preparation 2

An electrostatically spun polyetherurethane (BIOMER™) tube was modified as follows.

### 1.Functionalisation of the polyetherurethane.

The fibrous tube was reacted with trichloroacetyl isocyanate (3 g, 0.016 mole) in 150ml hexane for 24 hours. After this time the tube was washed off with water very carefully and subsequently immersed in water for 2 days and vacuum dried. The tube showed a positive chlorine test. The overall reaction of the polyetherurethane and trichloroacetyl isocyanate is depicted in the following equation:

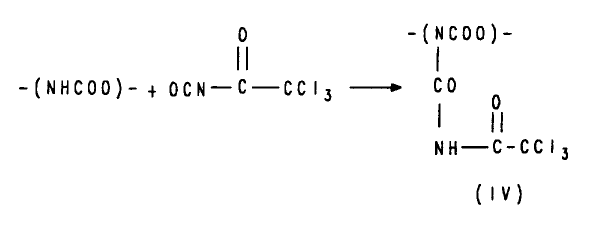

### 2.Grafting of N-acryloyloxysuccinimide

The fibrous tube of functionalized polyetherurethane (IV) was placed in a reaction vessel and a solution of Re₂(CO)₁₀ (0.095 g 0.00014 mole) and N-acryloyloxysuccinimide (0.75 g, 0.0044 mole) in 25 ml dry ethyl acetate was added. The reaction mixture was degassed under vacuum and the vessel sealed off. The reaction solution was photolysed at λ = 365nm at ambient temperature for 2 hours. Then the irradiation was continued under a 60 watt lamp for 24 hours with continuous rotation. The tube was then washed thoroughly with dry ethyl acetate and vacuum dried.

The chemical structure of the grafted polyetherurethane (V) is shown as follows:

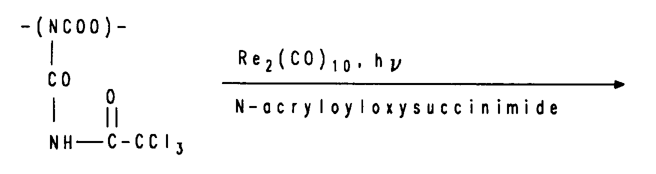

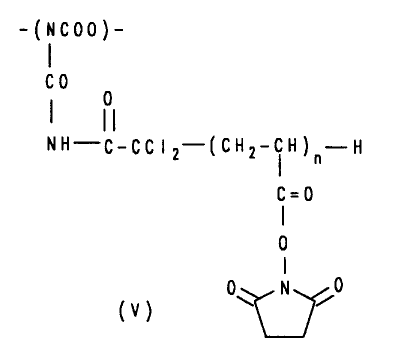

### Preparation 3

Polyetherurethane (BIOMER™) was dissolved in DMAC to form an 8% w/w solution. The solution was cast on a glass surface to produce a film. After evaporation of the solvent, the film was immersed in water and stripped from the glass surface. The film was washed extensively with water and dried.

The film of polyetherurethane was treated in a manner identical to that described for the fibrous tube in steps 1 and 2 of Preparation 2.

### Preparation 4

A sample of polyetherurethane (BIOMER™) was activated after electrostatic spinning by the method described below.

A sheet of the electrostatically spun polymer was immersed in hexane for one hour prior to addition of 10 g (an excess) of hexamethylene diisocyanate and the reaction was left standing at room temperature for 4 days. After this time the sheet was removed and carefully washed with hexane and vacuum dried. The reaction is illustrated as follows: 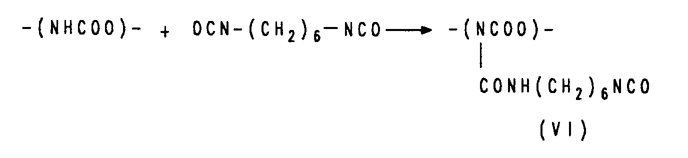
The sheet of polymer (VI) was placed in a flask containing a solution of 0.4 g of N-[3-(4-hydroxyphenyl)propionyloyl]succinimide (Fluka) in 40 ml of dry acetonitrile. The flask was wrapped in foil and stirred at room temperature for 5 days. After this time the sheet was removed and carefully washed with an excess of acetonitrile and vacuum dried. The activated polymer was produced according to the following equation. 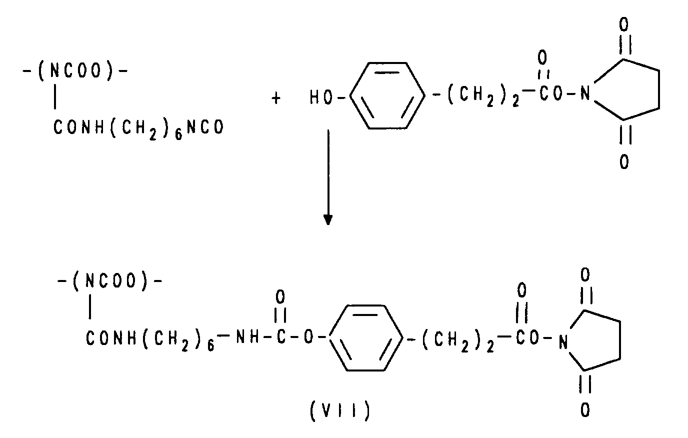
The above procedure was also carried out using 2,4-tolylene diisocyanate instead of hexamethylene diisocyanate to provide an activated polymer.

### Covalent coupling of protein

A solution of radiolabelled Protein A was prepared containing 1 mg Protein A/ml 0.1 molar sodium hydrogen carbonate, pH 8. A sample (2 ml) of the resulting solution was passed through a Millipore filter containing a disc (diameter = 2.54 cm) of the polymer (VII) at a flow rate of 1 ml/hour using a syringe-pump. After two hours the disc was removed and washed with 0.1 molar sodium hydrogen carbonate and deionised water. The disc was left standing for one hour in 10 ml sodium dodecyl sulphate (SDS) (2%), washed with deionised water and blotted dry. The disc was then counted for 1 minute in a vial using a scintillation counter. The results of the counting (CPM), the amount of protein A covalently bound to the polymer and also the amount of Protein A physically adsorbed on the unactivated polymer (BIOMER™) as a control are shown in Table 1 below.

**Table 1**

| Sample | Sample wt. (g) | CPM | Protein A mg/g | Protein A mg/m² |
|---|---|---|---|---|
| Protein A (I-125) | 0.0001 | 288,382 | | |
| Polymer (VII) | 0.1125 | 1,029,354 | 3.1728 | 704.42 |
| BIOMER™ | 0.1086 | 11,600 | 0.0370 | 7.93 |

### Preparation 5

A sample of polyetherurethane (BIOMER™) was activated after electrostatic spinning by the method described below.

The electrostatically spun polymer was reacted with isocyanatoethyl methacrylate monomer (20% v/v in hexane) at room temperature for 5 days. After this time the functionalized polyetherurethane was washed with hexane, methanol, water and methanol, respectively. The reaction is shown as follows. 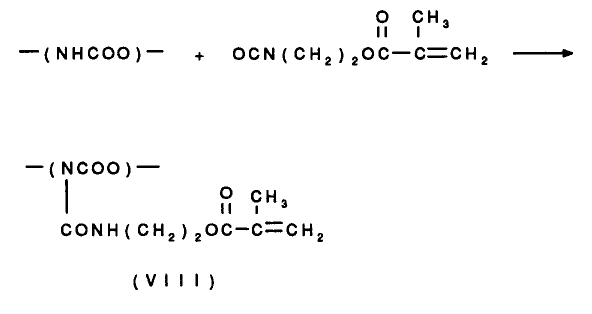
A specimen of macromer (VIII) (1.9 g) was placed in a reaction vessel containing a mixture of 0.5 g N-acryloyloxysuccinimide in 10 ml of dry acetonitrile and 0.2 g azobisisobutyronitrile (AIBN) dissolved in 10 ml acetonitrile. After degassing, the polymerization was carried out at 60°C for 4 hours. The macromer sheet was removed and washed with acetonitrile and vacuum dried. The resulting product (IX) was produced in accordance with the following reaction.

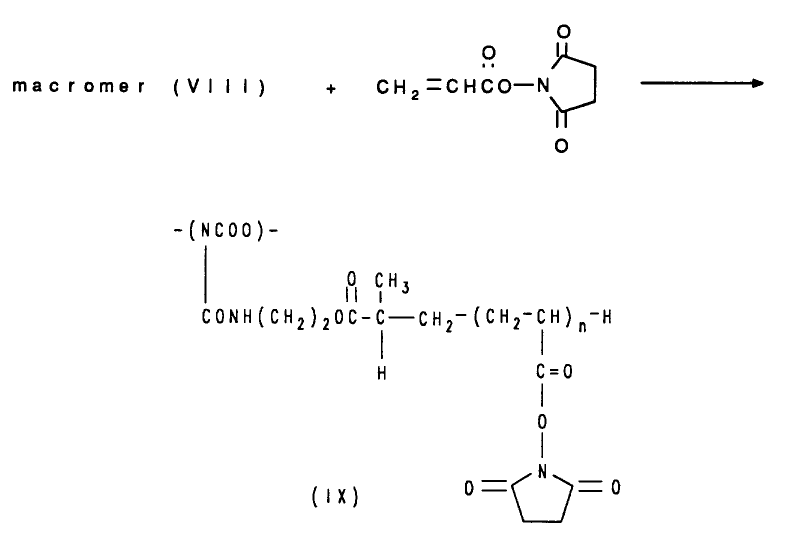

### Covalent coupling of protein

Protein A was bound to a sample of polymer (IX) following the procedure given in Preparation 4. The results are shown below in Table 2.

**Table 2**

| Sample | Sample Wt. (g) | CPM | Protein A mg/g | Protein A mg/m² |
|---|---|---|---|---|
| Protein A (I-125) | 0.0001 | 288,382 | | |
| Polymer(IX) | 0.1103 | 192,849 | 0.6062 | 132.00 |
| BIOMER™ | 0.1086 | 11,600 | 0.0370 | 7.93 |

### Preparation 6

A sample of polyetherurethane (BIOMER™) was activated after electrostatic spinning by the method described below.

The electrostatically spun polyetherurethane sheet was reacted with 2-chloroethyl isocyanate (1 g in 20 ml of hexane) for 24 hours at room temperature. After this time, the sheet was washed with hexane, methanol, water and methanol, respectively, and vacuum dried. A graft copolymer (X) was synthesized by grafting N-acryloyloxysuccinimide monomer (0.5 g in 10 ml acetonitrile) on to the chloroethyl isocyanated polyetherurethane in the presence of Re₂(C0)₁₀. The reaction is shown as follows.

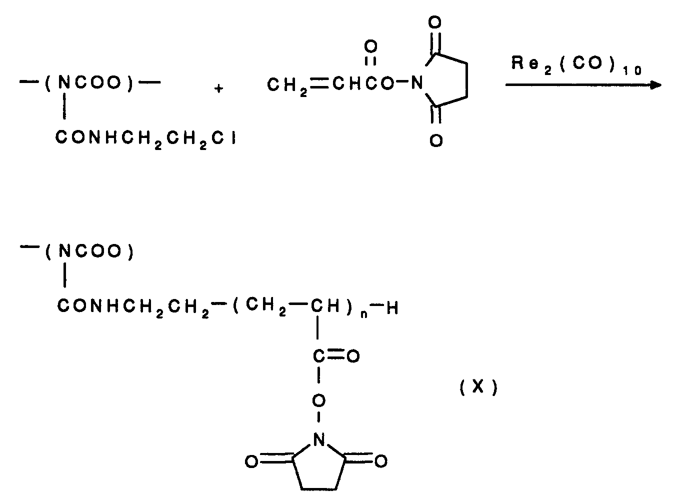

### Covalent coupling of protein

Protein A was bound to a sample of polymer (X) following the procedure given in Preparation 4. The results are shown below in Table 3.

**Table 3**

| Sample | Sample wt. (g) | CPM | Protein A mg/g | Protein A mg/m² |
|---|---|---|---|---|
| Protein A (I-125) | 0.0001 | 288,382 | | |
| Polymer (X) | 0.0843 | 403,335 | 1.6590 | 276.00 |
| BIOMER™ | 0.1086 | 11,600 | 0.0370 | 7.93 |

### Preparations 7 to 17

### Synthesis

Two samples of electrostatically spun BIOMER™ polymer sheet (2 g each) were placed in two reaction vessels. The first one was reacted with 30% hexamethylene diisocyanate in petroleum ether (b.p. 60-80°C) at 40°C, and the second reacted with bulk tolylene 2,4-diisocyanate at room temperature. The reaction time for these samples was five days, after which time the two samples were washed carefully with petroleum ether and vacuum dried.

Each of the isocyanated BIOMER™ polymer samples was reacted with bulk ethanolamine (25 ml) for 17 hours at room temperature to produce hydroxylated polymers.

One sample (1 g) of the hydroxylated BIOMER™ which had been isocyanated with hexamethylene diisocyanate was reacted with 0.5 g (1.7 mmole) of FMP dissolved in 10 ml of dry acetonitrile in the presence of triethylamine (0.2 ml) to give an activated polymer of the invention (Preparation 7). The reaction was carried out at room temperature for 24 hours. After this time, the sample was washed with dry acetonitrile and dried in a vacuum.

Another sample (1 g) of the same hydroxylated BIOMER™ polymer was reacted with CDI (0.5 g, 3 mmole) dissolved in acetonitrile to give an activated polymer of the invention (Preparation 8). The reaction was carried out at room temperature for 24 hours. After this time the sample was washed with dry acetonitrile and dried in a vacuum.

The same procedures as above were repeated for the activation of two samples of the hydroxylated BIOMER™ polymer which had been isocyanated with tolylene 2,4 diisocyanate. The sample activated with FMP gave an activated polymer of the invention (Preparation 9). Similarly, the sample activated with CDI gave an activated polymer of the invention (Preparation 10).

Following the procedures given above, other polyurethane samples isocyanated with hexamethylene diisocyanate and 2,4-tolylene diisocyanate, respectively, were converted to hydroxylic forms and activated with CDI. More particularly, polymer samples isocyanated with hexamethylene diisocyanate were converted to hydroxylic forms by reaction with 6-amino-1-hexanol (Preparation 11) and glucamine (Preparation 14). Polymer samples isocyanated with 2,4-tolylene diisocyanate were converted to hydroxylic forms by reaction with 6-amino-1-hexanol (Preparation 12), poly(ethylene glycol)( Molecular weight 4000) (Preparation 13) and glucamine (Preparation 15).

Carboxylated polymers were prepared from polyurethane samples isocyanated with hexamethylene diisocyanate and 2,4-tolylene diisocyanate, respectively. The isocyanated polymer samples were reacted with 6-aminocaproic acid and the resulting carboxylated polymers were activated using CDI (Preparations 16 and 17, respectively).

### Coupling of Protein A to activated BIOMER™ polymer

A disc of each of the polymers of Preparations 7 to 17 (diameter = 2.54 cm) was tested with Protein A solution. Each disc was placed in a Millipore filter holder and 3 ml of Protein A labeled with ¹²⁵I (1 mg/ml solution, prepared from Protein A ¹²⁵I (Amersham, 10 mCi) diluted with 20 mg of Protein A (Sigma) in 0.1 molar sodium hydrogen carbonate, pH 8) was passed through the disc at a flow rate of 1 ml/hour using a syringe pump. After three hours, each sample was washed extensively with 0.1 molar sodium hydrogen carbonate, followed by deionised water and then left standing in 10 ml of SDS (2%) for one hour. Each sample was then washed with deionised water, blotted dry and counted for one minute in a vial containing 8 ml of Optiphase scintillant. The result of counting (in cpm) and the amount of Protein A covalently bound to the activated supports together with a control are shown in Table 4 below.

**Table 4**

| Sample | Weight (g) | Activity (cpm) | Protein A(mg/g of BIOMER™) |
|---|---|---|---|
| Protein A | 0.0002 | 33399 | -- |
| Prepn. 7 | 0.13 | 143412 | 0.66 |
| Prepn. 8 | 0.0755 | 789072 | 6.26 |
| Prepn. 9 | 0.1043 | 51723 | 0.29 |
| Prepn. 10 | 0.1093 | 114798 | 0.63 |
| Prepn. 11 | 0.320 | ------ | 1.81 |
| Prepn. 12 | 0.306 | ------ | 1.46 |
| Prepn. 13 | 0.3032 | ------ | 1.43 |
| Prepn. 14 | 0.3159 | ------ | 2.94 |
| Prepn. 15 | 0.3494 | ------ | 3.04 |
| Prepn. 16 | 0.3083 | ------ | 3.64 |
| Prepn. 17 | 0.3280 | ------ | 4.16 |
| Control | 0.0918 | 2185 | 0.0143 |

### Preparation 18

### Protein A Coupling

Three discs (diameter = 2.54 cm) of polymer VII above and the polymer of Preparation 8 were each placed in a Millipore filter holder and 5 ml of protein A (1 mg/ml solution) in 0.1 molar sodium hydrogen carbonate (pH 8) passed through at a flow rate of 1 ml/hour using a syringe pump. After 5 hours, the samples were washed with 0.1 molar sodium hydrogen carbonate followed by water and blotted dry on filter paper. The samples were then reacted with blocking reagent (ethanolamine; pH 8; 10 ml) and left to stand for one hour at room temperature. The samples were washed with water and stored in PBS at 4°C. A similar procedure was carried out using unreacted BIOMER™ polymer as a control.

### IgG Binding

Three discs of each sample of protein A/polymer VII and protein A/polymer of Preparation 8 were placed in a Millipore filter holder and a solution of radiolabeled (labeled with ¹²⁵I) human IgG (2.8 mg/ml, 5 ml) in 0.15 molar PBS (pH 7) was passed through at a flow rate of 1 ml/hour. After 5 hours, the discs were removed, washed with water and placed in 0.15 molar PBS containing 0.2% TWEEN™ 20 nonionic surfactant for one hour to remove the non-specifically bound protein. The discs were washed with water and blotted dry on filter paper. Each disc was counted for one minute in 8 ml Optiphase scintillant. The results of counting (in cpm) and the amount of IgG bound to the protein A/polymer supports together with the results for the control are shown in Table 5 below.

**Table 5**

| Sample | Weight (g) | Activity (cpm) | IgG (mg/g polymer) |
|---|---|---|---|
| IgG (labeled) | 0.0005 | 243948 | -- |
| Polymer (VII) | 0.2876 | 338260 | 2.68 |
| IgG (labeled) | 0.0003 | 140527 | -- |
| Polymer (Prepn. 8) | 0.3713 | 744860 | 3.96 |
| Control | 0.3619 | 8613 | 0.02 |

### IgG Coupling

IgG was coupled to samples of polymer VII and the polymer of Preparation 8 directly. In this case three discs of each of these samples were placed in two Millipore filter holders and labeled IgG ¹²⁵I) (2 mg/ml, 5 ml) in PBS solution passed through at a flow rate of 1 ml/hour. After this time, the discs were removed, washed with water and placed in 10 ml of SDS (2%) for one hour. Each disc was then washed with water and counted for one minute in a vial containing 8 ml Optiphase scintillant. The results of IgG bound to the supports are shown in Table 6 below.

**Table 6**

| Sample | Weight (g) | Activity (cpm) | IgG (mg/g polymer) |
|---|---|---|---|
| IgG (labeled) | 0.0005 | 165237 | -- |
| Polymer VII | 0.2390 | 401526 | 5.65 |
| Polymer (Prepn.8) | 0.1834 | 310775 | 5.70 |
| Control | 0.2527 | 1456 | 0.019 |

Examples of immunoassay procedures in accordance with the invention are as follows.

### Example 1

### i) Activation

Five discs of BIOMER™ membrane were reacted with 40% hexamethylene diisocyanate in hexane for twentyfour hours at 40°C. The resulting isocyanated membrane was washed very carefully with dry acetonitrile and dried in vacuum before IgG coupling.

### ii) Coupling of IgG by perfusion

Human IgG (I-125), (4 ml of 1 mg/ml concentration in coupling buffer pH 8.4) was passed through each disc twice within two hours. After this time the discs were washed extensively with coupling buffer and subsequently with 0.2% Tween 20 in PBS before counting. The weights of IgG coupled are shown in Table 7.

**Table 7**

| Direct coupling of IgG to membrane | | |
|---|---|---|
| Sample Number | IgG coupled | |
| | mg/g Biomer | µg/cm² |
| 1 | 10.88 | 154.20 |
| 2 | 10.57 | 144.56 |
| Control | 0.11 | 1.54 |

### iii) Enzyme-linked immunoassay

Human IgG (unlabelled) was coupled to a sample of the activated membrane by the procedure described in (ii). Residual isocyanate groups were blocked by washing with a 1% solution of ethanolamine in 1 M sodium bicarbonate. The membrane was finally washed with PBS and dried by blotting with filter paper. It was then cut into discs (0.63 cm diameter) which were placed in a 96-well manifold. Further blocking was then carried out (to eliminate the possiblity of non-specific binding) by adding 200 µl of 1% v/v non-fatted dried milk in PBS to each well; this was followed by washing the discs with PBS and subsequently with 0.02% Tween solution in PBS.

### iv) Binding of anti-human IgG peroxidase conjugate

Two types of goat anti-Human IgG (F_{ab} and F_{c} specific) peroxidase conjugates were purchased from Sigma. Each was prepared in a series of dilutions - 1/100, 1/500, 1/2500 in 1% v/v non-fatted skimmed milk in PBS. 200 µl of each dilution were distributed between the wells of the manifold prepared as in (iii) and incubated for one hour at room temperature. The discs were then washed very extensively with 0.02% Tween in PBS. Chromogen (substrate ABTS) was added, 200 µl in each well. After fifteen minutes at room temperature, the blue-green colour developed was assessed by ELISA reader.

A precisely similar series of experiments was carried out with a commercially available micrporous membrane, IMMOBILON™ (Millipore).

With the polyurethane membrane the lower dilutions of anti-Human IgG (1/100, 1/500) rapidly gave colours too intense to measure, while with the commercial material colour development was slower. With the highest dilution (1/2500) the absorbance using the polyurethane membrane was 1.81 and in IMMOBILON™ 1.1.

Controls with anti-Human IgG peroxides, without Human IgG gave no significant colour in any test.

### Example 2

### (i) Diffusional binding

Human IgG (unlabelled) was coupled to four discs (d=2.54cm, each) of the activated BIOMER™ membrane described in Example 1 by the procedure described therein. The unreacted isocyanate groups were blocked by washing with 1% solution of ethanolamine in 1M sodium bicarbonate.

The discs were washed with 0.2% Tween in PBS and finally with PBS. Further blocking was then carried out (to eliminate the possibility of non-specific binding) by adding 1.2ml of 1% v/v non-fatted dried milk in PBS to each disc; this was followed by washing the discs with 0.02% Tween solution in PBS.

Two types of goat anti-human IgG peroxidase (AHIP) conjugates (F_{ab} and F_{c} specific) were prepared in two dilutions of 1/2500 and 1/5000 in 1% v/v non-fatted skimmed milk in PBS. 1.2ml of each dilution were added on each disc of IgG coupled membrane and incubated for one hour at room temperature. The discs were then washed extensively with 60ml of 0.2% Tween in PBS by perfusion method. From each disc was then cut three discs (0.63cm diameter) which were placed in a 96-well manifold for ELISA reader. Chromogen (substrate ABTS) was added, 200µl in each well. After fifteen minutes at room temperature, the absorbance (405nm) of green colour developed was measured by ELISA reader. Control discs of unactivated Biomer were incubated with goat anti-human IgG peroxidase, without human IgG treated similarly as above and tested by ELISA technique. The absorbance results are shown in Table 8.

**Table 8**

| Sensitivity of detection of human IgG coupled to BIOMER™ membrane | | |
|---|---|---|
| AHIP dilution | Absorbance (405nm) from BIOMER-IgG membrane | Absorbance (405nm) from BIOMER control membrane |
| F_{ab} 1/2500 | 0.692,0.773,0.718 | 0.004,0.003,0.004 |
| F_{ab} 1/5000 | 0.460,0.404,0.439 | 0.005,0.007,0.010 |
| F_{c} 1/2500 | 0.654,0.695,0.890 | 0.010,0.015,0.016 |
| F_{c} 1/5000 | 0.340,0.207,0.240 | 0.011,0.010,0.010 |

### (ii) Dot binding

One disc (d=2.54cm) of the activated BIOMER™ membrane was exposed by dot binding mode to 400µl of human IgG (1mg/ml in coupling buffer pH 8.5) for one hour at room temperature. Remaining isocyanated groups were then reacted with 1% v/v ethanolamine in 1M sodium bicarbonate and the disc washed with 0.2% Tween in PBS and with PBS respectively. The disc was then exposed by dot binding to 400µl of AHIP (F_{ab} specific) of 1/100 dilution in 1% non-fatted skimmed milk for one hour at room temperature and washed with 0.2% Tween in PBS and finally with PBS.

A control disc of unactivated BIOMER membrane was exposed to 400µl of AHIP without human IgG and washed as above.

Chromogen (substrate ABTS) was added, 2ml on each disc and incubated for 15 minutes. A positive antibody reaction was observed after this time as dark purple spot against the white background of the control.

### Example 3

### Poly(ether-urethane) membrane for Enzyme-linked Immunoassay (Full Sandwich Assay)

The detection of human tumor necrosis factor alpha (TNF_{α}) antigen was carried out by a full sandwich immunoassay. The key steps for such assay are as follows.

### Method

(1) A disc (d=2.54cm) of the activated BIOMER™ membrane described in Example 1 was reacted with a solution of 200µg mouse monoclonal antibody IgG1 (JID9) in 2ml coupling buffer (pH 9.2)(0.1m sodium phosphate and 0.15m sodium chloride). The reaction of JID9 with the membrane was carried out by perfusion for 2 hours at room temperature and then followed by immersion for 17 hours at 4°C. The disc was then washed very carefully with 0.2% Tween in PBS and finally with PBS.
(2) The unreacted isocyanated groups were blocked with 1% solution of ethanolamine in 1M sodium bicarbonate and subsequently with a solution of 1% v/v non-fatted dried milk in PBS; this was followed by washing the disc with 0.02% Tween solution in PBS.
(3) The membrane to which JID9 had been coupled was immersed in 2ml PBS containing 5µg human TNF_{α} (specific activity 6 x 10⁷ U/mg) for two hours at room temperature. The membrane was then removed and washed with 0.2% Tween in PBS and finally with PBS.
(4) The membrane having TNF antigen immobilised thereon was fully exposed to rabbit anti-mouse IgG polyclonal antibody (dilution of 1/100 in PBS-Tween) and allowed to bind at room temperature for one hour. The disc was washed very carefully with 0.2% Tween in PBS and PBS, respectively.
(5) The membrane-bound polyclonal antibody was incubated with peroxidase anti-rabbit IgG (ARIP)(produced by Silenus) diluted in 1/5000 in PBS-Tween and allowed to bind at room temperature for one hour. The disc was washed with 0.2% Tween-PBS and finally with PBS.
(6) From the resulting membrane, four discs (d=0.63cm) were cut which were then placed in 96-well plate and incubated with 200µl chromogen of 0.3mg/ml 2,2-azino-bis (3-ethylbenzenthiazoline sulfonic acid) (ABTS) in 0.1M citrate buffer PH4.0 containing 0.02% H₂O₂. The absorbance at 405nm of the green colour solution developed was measured by ELISA reader. The absorbance data are shown in Table 9.
(7) A control disc of unactivated BIOMER™ membrane was treated as in steps (1), (5) and (6) above to determine the non-specific adsorption of antibodies and the absorbance data are shown in Table 9.

**Table 9**

| Detection of human antigen TNF on to activated BIOMER™ membrane (1) immobilized with monoclonal antibody JID9. | |
|---|---|
| | Absorbance at 405nm |
| BIOMER membrane-JID9 | 0.296,0.329,0.381,0.349 |
| unactivated BIOMER membrane as control | 0.020,0.007,0.014,0.008 |

### Materials

Recombinant human TNF, monoclonal antibody JID9 and polyclonal antibody were obtained from Dr P J McLaughlin, Department of Immunology, Liverpool University

## Claims

1. An immunoassay for the detection of an analyte which comprises
(A) forming a detectably labeled immune complex comprising the analyte on a support, and
(B) detecting the labeled immune complex on the support as an indication of the presence or absence of the analyte,
characterised in that the support is derived from a fibre-forming or film-forming polyamide, polyurethane or polyurea polymer comprising activating groups attached to said polymer through the nitrogen atom of the amide, urethane or urea groups of said polymer, the activating groups being capable of reaction with an amino or thiol group of a compound containing an amino or thiol group to effect covalent attachment of the compound to the polymer and the detectably labeled immune complex is covalently bound to the polymer by the formation of a link by reaction between the activating group of the polymer and a component of the immune complex.

2. An immunoassay according to claim 1 wherein the support is a porous membrane having a pore size of from 0.5 to 10 µm.

3. An immunoassay according to claim 1 or claim 2 wherein the support is fibrous.

4. An immunoassay according to any one of the preceding claims wherein the support is derived from a polyetherurethane.

5. An immunoassay according to any one of the preceding claims comprising the steps of
(i) contacting an aqueous specimen suspected of containing the analyte with the support having an anti-analyte antibody covalently bound thereto, and
(ii) prior to, simultaneously with or subsequent to the contact in step (i), labelling the analyte with a detectably labelled antibody.

6. An immunoassay according to claim 5 wherein the detectably labelled antibody is an enzymelabelled antibody.

7. A test kit for an immunoassay for the detection of an analyte comprising:
(a) a water-insoluble article comprising a porous membrane having at least first and second opposing outer surfaces and having an anti-analyte antibody covalently bound thereto, and
(b) detectably labelled antibodies directed to the analyte,
the kit characterised in that the porous membrane is derived from a fibre-forming or film-forming polyamide, polyurethane or polyurea polymer comprising activating groups attached to said polymer through the nitrogen atom of the amide, urethane or urea groups of said polymer, the activating groups being capable of reaction with an amino or thiol group of a compound containing an amino or thiol group to effect covalent attachment of the compound to the polymer and the anti-analyte antibody is covalently bound to the polymer by the formation of a link by reaction between the activating group of the polymer and the antibody.
